# EUROPEAN PATENT SPECIFICATION

(11) **EP 2 760 581 B1**
(45) Date of publication and mention of the grant of the patent: **09.11.2016**
(21) Application number: 12781456.4
(22) Date of filing: 23.09.2012
(51) Int. Cl.: B01J 31/24, C07F 15/00, C08F 8/48

(54) **RUTHENIUM COMPLEX, METHOD FOR ITS PREPARATION AND USE THEREOF**
RUTHENIUMKOMPLEX, VERFAHREN ZUR HERSTELLUNG SOWIE VERWENDUNG
COMPLEXE DU RUTHÉNIUM, PROCÉDÉ POUR SA PRÉPARATION ET SON UTILISATION

(30) Priority: 26.09.2011 PL 39643911
(43) Date of publication of application: 06.08.2014
(73) Proprietor: Instytut Chemii Organicznej Polskiej Akademii Nauk, 01-224 Warszawa (PL)
(72) Inventor: WDOWIK, Tomasz, PL-35-113 Rzeszów (PL); SAMOJLOWICZ, Cezary, PL-02-786PL Warszawa (PL); JAWICZUK, Magdalena, PL-21-560 Miedzyrzecz Podlaski (PL); GRELA, Karol, PL-01-471 Warszawa (PL)
(74) Representative: Witek, Rafal
(86) International application number: PCT/IB2012/055058
(87) International publication number: WO 2013/046108

(56) References cited:
- WAI YIU HUNG ET AL: "Electrophilic Substitution Reactions of Metallabenzynes", JOURNAL OF THE AMERICAN CHEMICAL SOCIETY, vol. 133, no. 45, 16 November 2011 (2011-11-16), pages 18350-18360, XP055045798, ISSN: 0002-7863, DOI: 10.1021/ja207315h
- JOSEPH N. COALTER ET AL: "Reactivity of [RuHCl(P iPr3)2] 2 with Functionalized Vinyl Substrates. The H2 Ligand as a Sensitive Probe of Electronic Structure", INORGANIC CHEMISTRY, vol. 39, no. 17, 1 August 2000 (2000-08-01), pages 3749-3756, XP055045907, ISSN: 0020-1669, DOI: 10.1021/ic9905998
- RAFAL GAWIN ET AL: "A Dormant Ruthenium Catalyst Bearing a Chelating Carboxylate Ligand: In-Situ Activation and Application in Metathesis Reactions", ANGEWANDTE CHEMIE INTERNATIONAL EDITION, vol. 46, no. 38, 24 September 2007 (2007-09-24), pages 7206-7209, XP055045928, ISSN: 1433-7851, DOI: 10.1002/anie.200701302 cited in the application

## Description

The present invention relates to novel complexes of metals that act as pre(catalysts), a method of preparation them as well as their use in the metathesis, isomerisation and cycloisomerisation of olefins, and cycloisomerisation reactions, in olefin and in hydrogen transfer. The present invention is useful in broadly understood organic synthesis.

The use of olefin metathesis in organic synthesis has recently seen much progress. The state of the art reveals several carbene complexes of ruthenium acting as (pre)catalysts which possess both high activity in metathesis reactions of various kinds, as well as a broad tolerance of functional groups. The above combination of properties warrants the utility of these types of (pre)catalysts in organic synthesis.

From the point of view of practical use, particularly on an industrial scale, it is very desirable that such ruthenium complexes are stable, for extended periods at elevated temperatures, and may be stored and/or purified and/or used without a protective gas atmosphere. It is also important that these catalysts exhibit variable reactivity, depending on the reaction conditions, and that they are easy to remove after the reaction.

Many complexes of ruthenium active in olefin metathesis have been disclosed (see: Org. Lett. 1999, 1, 953-956; J. Chem. Soc. Chem. Commun. 1999, 601-602). It is also known that increased stability is connected with decreased catalytic activity (for comparison: J. Amer. Chem. Soc. 2000, 122, 8168-8179; Tetrahedron Lett. 2000, 41, 9973-9976). These types of advantages and limitathions have also been noted i the case of (pre)catalysts activated by steric or electron factors of the benzylidene ligands (for a comparison of catalytic activity see: Angew. Chem. Int. Ed. 2002, 114, 4210-4212; Angew. Chem. Int. Ed. 2002, 114, 2403-2405). The osmium complex has been disclosed in Hung et al., J.Am.Chem.Soc., 2011, 133(45), 18350-60 which refers to the electrophilic substitution reactions of metallabenzynes. The metallabenzynes react with electrophilic reagents to afford the bromination, nitration, nitrosation and chlorination products. Coalter et al., Inorg. Chem., 2000, 39(17), 3749-56 presented a reactivity of ruthenium complex with functionalized vinyl substrates. Gawin et al., Angew. Chem. Int. Ed., 2007, 46(38),7206-9 discloses a number of ruthenium catalysts which contain a chelating 2-alkoxybenzylidene group.

The effect of anionic ligands has also been demonstrated (see: Angew. Chem. Int. Ed. 2007, 46, 7206-7209; Organometallics, 2010, 29, 6045-6050; Organometallics, 2011, 30, 3971-3980) as well as of NHC ligands (see: Chem. Rev. 2010, 110, 1746-1787; Chem. Rev. 2009, 109, 3708-3742) on the activity and selectivity of (pre)catalysts. From these reports, it stems that the exchange of a chloride ligand for an oxyacid residue increases the stability of the (pre)catalyst.

Unexpectedly it was shown that the novel ruthenium complexes according to the present invention defined by Formula 1: 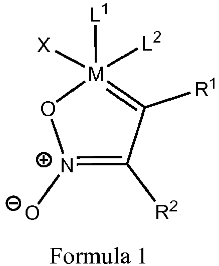 which contains a chelate ring formed by an oxygen atom are thermally stable and exhibit good catalytic activity. Additionally, these compounds significantly alter the selectivity of the reaction depending on the use of: a solvent and/or the addition of an acid or halide derivatives of alkanes or halide derivatives of silanes or *N*-haloimides or *N*-haloamides; which enables the control over the catalytic processes through the exchange of these factors.

Complexes defined by Formula **1,** according to the present invention are useful in a broad range of reactions. A good result may be obtained by conducting both numerous metathesis ring closure reactions, homometatheses, cross metatheses as well as well as metatheses of the "alkene-alkyne" (ene-yne), ring-opening polymerisation reactions (ROMP), olefin isomerisation reactions, olefin cycloisomerisation reactions as well as hydrogen transfer reactions.

The high polarity of the compounds being the subject of the present invention also makes it easier to remove ruthenium compounds from the reaction products, which is very significant in the synthesis of compounds for the pharmaceutical industry.

The subject of the present invention is a metal complex defined by Formula **1**: 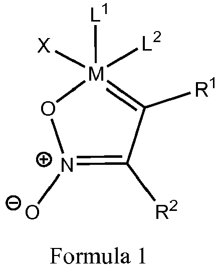 in which
M denotes ruthenium;
X denotes a chlorine atom;
R¹ denotes a hydrogen atom, methyl group;
R² denotes a hydrogen atom;
neutral ligand L¹ denotes -P(R⁵)₃ in which substituent R⁵ denotes, independently of one another, C₁-C₁₂ alkyl, C₃-C₁₂ cycloalkyl, C₅-C₂₀ aryl, 5-12 membered heteroaryl; and neutral ligand L² denotes ligands defined by Formula **2a** or **2b**: 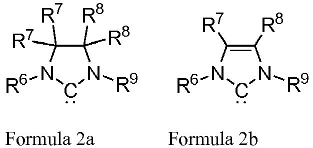 in which substituents R⁶, R⁷, R⁸ and R⁹ denotes, independently of one another, a hydrogen atom, C₁-C₁₂ alkyl, C₃-C₁₂ cycloalkyl, C₂-C₁₂ alkenyl or C₅-C₂₀ aryl which may be substituted with at least one C₁-C₁₂ alkyl, C₁-C₁₂ perfluoroalkyl, C₁-C₁₂ alkoxyl or halogen atom, and groups R⁶, R⁷, R⁸, R⁹ and R¹⁰ may possibly be interconnected.

Further subject of the invention is a method of producing a the ruthenium complex defined in Claim 1, characterised in that the compound defined by Formula **3** 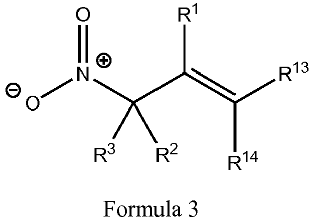
in which R¹, R² denote, independently of one another, a hydrogen atom, a fluoride atom, C₁-C₂₅ alkyl, C₁-C₂₅ perfluoroalkyl, C₂-C₂₅ alkene, C₃-C₇ cycloalkyl, C₂-C₂₅ alkenyl, C₁-C₂₅ cycloalkenyl, C₂-C₂₅ alkynyl, C₁-C₂₅ cycloalkynyl, C₁-C₂₅ alkoxyl, C₅-C₂₄ aryl, heteroaryl C₅-C₂₀, or a 3-12 membered heterocycle wherein the alkyl groups may be joined together in a ring, preferentially a hydrogen, a nitro group (-NO₂), a cyanide group (-CN), carboxyl (-COOH), ester (-COOR'), amido (-CONR^{'}₂), sulphonyl (-SO₂R^{'}), formyl (-CHO), sulphonoamido (-SO₂NR'₂), ketone (-COR'), in which R has the following meaning: C₁-C₅ alkyl, C₁-C₅ perfluoroalkyl, C₅-C₂₄ aryl, whereas R³, R¹³ R¹⁴ denote, independently of one another, a hydrogen atom, a fluoride atom, a C₁-C₂₅ alkyl, C₁-C₂₅ perfluoroalkyl, C₂-C₂₅ alkene, C₃-C₇ cycloalkyl, C₂-C₂₅ alkenyl, C₃-C₂₅ cycloalkenyl, C₂-C₂₅ alkynyl, C₃-C₂₅ cycloalkynyl, C₁-C₂₅ alkoxyl, C₅-C₂₄ aryl, heteroaryl C₅-C₂₀, or a 3-12 membered heterocycle wherein the alkyl groups may be joined together in a ring, preferentially a hydrogen, a nitro group (-NO₂), a cyanide group (-CN), a carboxyl (-COOH), ester (-COOR'), amido (-CONR'₂), sulphonyl (-SO₂R^{'}), formyl (-CHO), sulphonoamido (-SO₂NR'₂), or ketone (-COR') group, in which R has the following meaning: C₁-C₅ alkyl, C₁-C₅ perfluoroalkyl or C₅-C₂₄ aryl;
is reacted with carbene complexes of ruthenium defined by Formula **4a, 4b, 4c or 4d**: 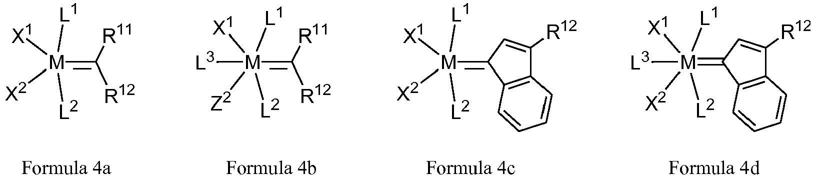 in which
   M denotes ruthenium or osmium;
   L¹, L² and L³, independently of one another, denote neutral ligands;
   X¹ and X², independently of one another, denote an anionic ligand;
   R¹¹ has the same meaning as R¹ of Formula **1**;
   R¹² denotes a hydrogen atom, C₅-C₂₀ aryl, C₅-C₂₀ heteroaryl, vinyl or allenyl.

Preferentially, the reaction is carried out over a period from 1 min. do 250 h, at a temperature in the range from 0 to 150°C.

Preferentially, the reaction is carried out in a chlorinated solvent or in aromatic hydrocarbons, or in protic or aprotic solvents, such as alcohols or ketones or in mixtures thereof.

Preferentially, the reaction is carried out in a solvent selected from among methylene chloride and/or toluene.

The present invention also relates to the use of complexes of ruthenium defined by Formula **1** as (pre)catalysts in metathesis reactions.

Preferentially, ruthenium complexes defined by Formula **1** are used as (pre)catalysts in metathesis ring closing reactions, homometatheses, cross-metatheses, "alkene-alkyne" metathesis (ene-yne) ROMP polymerisations as well as isomerisation and cyclomerisation of olefin and in the hydrogen transfer reactions.

Preferentially, complexes of ruthenium are used as (pre)catalysts in a metathetic polymerisation with dicyclopentadiene ring opening.

Preferentially, the reaction is carried out in the presence of an acid or halide derivatives of alkanes and silanes or *N*-haloimides and amides.

The term "a fluoride atom" denotes an element selected from among F, Cl, Br, or I.

The term "carbene" denotes a molecule containing a neutral carbon atom with a valence number of two and two unpaired valence electrons. The term "carbene" also encompasses carbene analogues in which the carbon atom is substituted by another chemical elements such as boron, silicon, germanium, tin, lead, nitrogen, phosphorus, sulphur, selenium and tellurium.

The term "alkyl" refers to a saturated, linear, or branched hydrocarbon substituent with the indicated number of carbon atoms. Examples of an alkyl substituent are -methyl, -ethyl, -*n-*propyl, -*n*-butyl, -*n*-pentyl, -*n*-hexyl, -*n*-heptyl, -*n*-octyl, -*n*-nonyl, and -*n*-decyl. Representative branched -(C₁-C₁₀)alkyls encompass -isopropyl, *-sec*-butyl, -isobutyl, *-tert*-butyl, -isopentyl, - neopentyl, -1-methylbutyl, -2-methylbutyl, -3-methylbutyl, -1,1-dimethylpropyl, -1,2-dimethylpropyl, -1-methylpentyl, -2-methylpentyl, -3-methylpentyl, -4-methylpentyl, -1-ethylbutyl, -2-ethylbutyl, -3-ethylbutyl, -1,1-dimethylbutyl, -1,2-dimethylbutyl, -1,3-dimethylbutyl, -2,2-dimethylbutyl, -2,3-dimethylbutyl, -3,3-dimethylbutyl, -1-methylhexyl, -2-methylhexyl, -3-methylhexyl, -4-methylhexyl, -5-methylhexyl, -1,2-dimethylpentyl, -1,3-dimethylpentyl, -1,2-dimethylhexyl, -1,3-dimethylhexyl, -3,3-dimethylhexyl, -1,2-dimethylheptyl, -1,3-dimethylheptyl, and -3,3-dimethylheptyl and the like.

Preferentially, ruthenium complexes defined by Formula **1** are used as (pre)catalysts in metathesis ring closing reactions, homometatheses, cross-metatheses, "alkene-alkyne" metathesis (ene-yne) ROMP polymerisations as well as olefin cyclomerisation reactions..

The term "a fluoride atom" denotes an element selected from among F, Cl, Br, or I.

The term "carbene" denotes a molecule containing a neutral carbon atom with a valence number of two and two unpaired valence electrons. The term "carbene" also encompasses carbene analogues in which the carbon atom is substituted by another chemical elements such as boron, silicon, germanium, tin, lead, nitrogen, phosphorus, sulphur, selenium and tellurium.

The term "alkyl" refers to a saturated, linear, or branched hydrocarbon substituent with the indicated number of carbon atoms. Examples of an alkyl substituent are -methyl, -ethyl, -*n-*propyl, -*n*-butyl, -*n*-pentyl, -*n*-hexyl, -*n*-heptyl, -*n*-octyl, -*n*-nonyl, and -*n*-decyl. Representative branched -(C₁-C₁₀)alkyls encompass -isopropyl, -*sec*-butyl, -isobutyl, *-tert*-butyl, -isopentyl, - neopentyl, -1-methylbutyl, -2-methylbutyl, -3-methylbutyl, -1,1-dimethylpropyl, -1,2-dimethylpropyl, -1-methylpentyl, -2-methylpentyl, -3-methylpentyl, -4-methylpentyl, -1-ethylbutyl, -2-ethylbutyl, -3-ethylbutyl, -1,1-dimethylbutyl, -1,2-dimethylbutyl, -1,3-dimethylbutyl, -2,2-dimethylbutyl, -2,3-dimethylbutyl, -3,3-dimethylbutyl, -1-methylhexyl, -2-methylhexyl, -3-methylhexyl, -4-methylhexyl, -5-methylhexyl, -1,2-dimethylpentyl, -1,3-dimethylpentyl, -1,2-dimethylhexyl, -1,3-dimethylhexyl, -3,3-dimethylhexyl, -1,2-dimethylheptyl, -1,3-dimethylheptyl, and -3,3-dimethylheptyl and the like.

The term "alkoxyl" refers to an alkyl substituent as defined above attached via an oxygen atom.

The term "perfluoroalkyl" denotes an alkyl group as defined above in which all hydrogen atoms have been replaced by identical or different atomy fluoride atoms.

The term "cycloalkyl" refers to a saturated mono- or polycyclic hydrocarbon substituent with the indicated number of carbon atoms. Examples of cycloalkyl substituents are -cyclopropyl, - cyclobutyl, -cyclopentyl, -cyclohexyl, -cycloheptyl, -cyclooctyl, -cyclononyl, -cyclodecyl, and the like.

The term "alkenyl" refers to an unsaturated, linear, or branched acyclic hydrocarbon substituent with the indicated number of carbon atoms and containing at least one double carbon-carbon bond. Examples of alkenyl substituents are: -vinyl, -allyl, -1-butenyl, -2-butenyl, - isobutylenyl, -1-pentenyl, -2-pentenyl, -3-methyl-1-butenyl, -2-methyl-2-butenyl, -2,3-dimethyl-2-butenyl, -1-hexenyl, -2-hexenyl, -3-hexenyl, -1-heptenyl, -2-heptenyl, -3-heptenyl, -1-octenyl, - 2-octenyl, -3-octenyl, -1-nonenyl, -2-nonenyl, -3-nonenyl, -1-decenyl, -2-decenyl, -3-decenyl and the like.

The term "cycloalkenyl" refers to an unsaturated mono- or polycyclic hydrocarbon substituent with the indicated number of carbon atoms and containing at least one double carbon-carbon bond. Examples of cycloalkenyl substituents are -cyclopentenyl, -cyclopentadienyl, - cyclohexenyl, -cyclohexadienyl, -cycloheptenyl, -cycloheptadienyl, -cycloheptatrienyl, -cyclooctenyl, -cyclooctadienyl, -cyclooctatrienyl, -cyclooctatetraenyl, -cyclononenyl, - cyclononadienyl, -cyclodecenyl, -cyclodekadienyl and the like.

The term "alkynyl" refers to an unsaturated, linear, or branched acyclic hydrocarbon substituent with the indicated number of carbon atoms and containing at least one triple carbon-carbon bond. Examples of alkynyl substituents are -acethylenyl, -propynyl, -1-butynyl, -2-butynyl, -1-pentynyl, -2-pentynyl, -3-methyl-1-butynyl, -4-pentynyl, -1-hexynyl, -2-hexynyl, -5-hexynyl and the like.

The term "cycloalkynyl" refers to saturated mono- or polycyclic hydrocarbon substituent with the indicated number of carbon atoms and containing at least one triple carbon-carbon bond. Examples of cycloalkynyl substituents are -cyclohexynyl, -cycloheptynyl, -cyclooctynyl, and the like.

The term "aryl" refers to an aromatic mono- or polycyclic hydrocarbon substituent with the indicated number of carbon atoms. Examples of aryl substituents are -phenyl, -tolyl, -xylyl, - naphthyl and the like.

The term "heteroaryl" refers to an aromatic mono- or polycyclic hydrocarbon substituent with the indicated number of carbon atoms in which at least one the carbon atom has been replaced by a heteroatom selected from among O, N and S. Examples of heteroaryl substituents are -furyl,-thienyl,- imidazolyl, -oxazolyl, -thiazolyl, -isoxazolyl, -triazolyl, -oxadiazolyl, -thiadiazolyl, - tetrazolyl, -pirydyl, -pirymidyl, -triazynyl, -indolyl, -benzo[b]furyl, -benzo[b]thienyl, -indazolyl, -benzoimidazolyl, -azaindolyl, -quinolyl, -isoquinolyl, -carbazolyl and the like.

The term "heterocycle" refers to saturated or partially unsaturated mono- or polycyclic hydrocarbon substituents, with the indicated number of carbon atoms in which at least one the carbon atom has been replaced by heteroatom selected from among O, N and S. Examples of heterocyclic substituents are -furyl, -thiophenyl, -pyrolyl, -oxazolyl, -imidazolyl, -thiazolyl, - isoxazolyl, -pirazolyl, -isothiazolyl, -triazynyl, -pyrolidynonyl, -pyrolidynyl, -hydantoinyl, - oxiranyl, -oxethanyl, -tetrahydrofuranyl, -tetrahydrothiophenyl, -quinolinyl, -isoquinolinyl, - chromonyl, -cumarynyl, -indolyl, -indolizynyl, -benzo[b]furanyl, -benzo[b]thiophenyl, - indazolyl, -purynyl, -4*H-*quinolizynyl, -isoquinolyl, -quinolyl, -phthalazynyl, -naphthyrydynyl, - carbazolyl, -β-carbolinyl and the like.

The term "neutral ligands" refers to uncharged substituents, capable of coordinating with a metallic centre (ruthenium atom). Examples of such ligands may be: amines, phosphines and their oxides, alkyl and alkane phosphorines and phosphoranes, arsines and their oxides, ethers, alkyl and aryl sulphides, coordinated hydrocarbons, alkyl and aryl halides.

The term "indenyl" refers to an unsaturated hydrocarbon substituent with an inden skeleton (benzocyclopentadiene).

The term "heteroindenyl" refers to an indenyl substituent, defined above in which at least one carbon atom is replaced with a heteroatom from a group encompassing: nitrogen, oxygen and sulphur.

The term "an anionic ligand" refers to a substituent capable of coordinating with a metallic centre (ruthenium atom) possessing a charge capable of the partial or full compensation of the metallic centre charge. Examples of such ligands may be: fluoride, chloride, bromide, iodide, cianide, cyanin and thiocyanin anions, carboxylic acid anions, alcohol anions, anions of phenols, thiols and thiophenols, anions of hydrocarbons with a displaced charge (i.e. cyclopentadiene), anions of (organo)sulphuric and (organo)phosphoric acids as well as their esters (such as i.e. anions of alkylsulphonic and arylsulphonic acids, anions of alkylphosphoric and arylphosphoric acids, anions of alkyl and aryl esters of sulphuric acid, anions of alkyl and aryl estersof phosphoric acids, anions of alkyl and aryl esters of alkylphosphoric and arylphosphoric acids). Possibly, an anionic ligand may possess linked L¹, L², L³ groups such as a katechol anion, an acetylacetone anion, a salicylic aldehyde anion. Anionic ligands (X¹, X²) as well as neutral ligands (L¹, L², L³) may be linked forming polydentate ligands, for example: bidentate ligands (X¹, X²), tridentate ligands (X¹, X², L¹), tetradentate ligands (X¹, X², L¹, L²), bidentate ligands (X¹, L¹), tridentate ligands (X¹, L¹ L²), tetradentate ligands (X¹, L¹, L², L³), bidentate ligands (L¹, L²), tridentate ligands (L¹, L², L³). Examples of such ligands are: a katechol anion, an aceylacetone anion as well as a salicylic aldehyde anion.

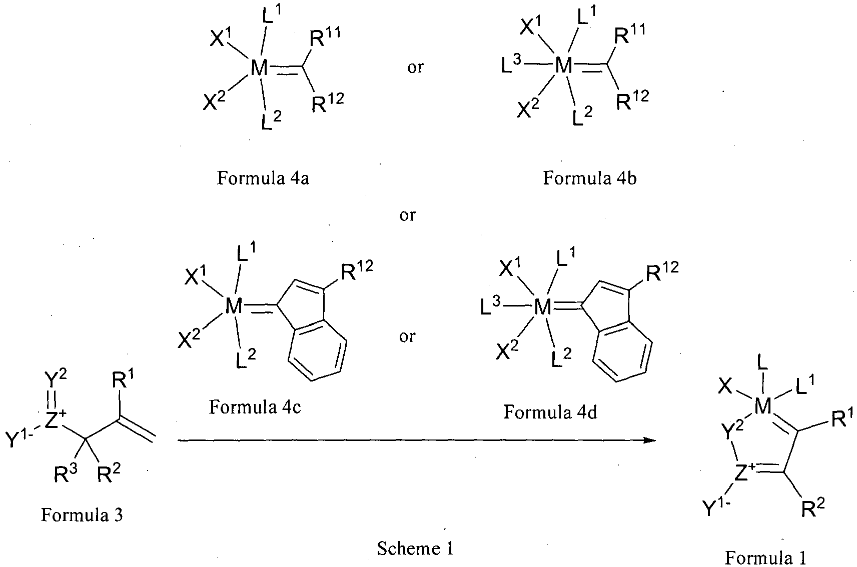

The examples below explain the production and use of the novel complexes.

### Example I:

### Synthesis of a catalyst defined by Formula la (according to Scheme I)

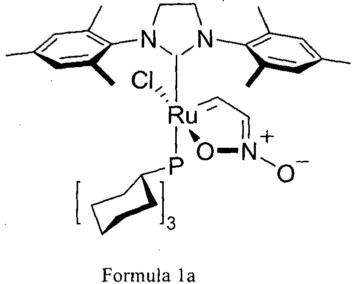

Using a protective argon atmosphere in a Schlenk vessel, we placed a solid carbene metal complex defined by Formula **4a,** in which M denotes ruthenium, X¹ and X² denote chlorine, L¹ denotes tricyclohexylphosphine (PCy₃), L² denotes the NHC ligands defined by Formula **2a,** in which R⁶ and R⁹ denote 2,4,6-trimethylphenyl, R⁷, R⁸ as well as R¹¹ are hydrogen and R¹² is phenyl (so-called Grubbs II-generation catalyst, 102 mg, 0.12 mmol), we added dry deoxygenated dichloromethane (2 ml). Next, we added the compound defined by Formula 3a: 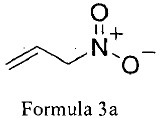 (13.1 mg, 0.15 mmol). The resulting solution were mixed at room temperature for 20 hours. From this time, all subsequent operations were performed in the open air, without the need for a protective argon atmosphere. The reaction mixture was concentrated in an evaporator and loaded onto a chromatography column packed with a silica gel. The column was developed with an ethyl acetate-cyclohexane solution (10% v/v), collecting the green fraction. After evaporating off the solvent, we obtained complex **1a** as an olive, microcrystalline solid (52.6 mg, 55% efficiency).
¹H NMR (500 MHz, CDCl₃): d = 14.27 (d, J = 3 Hz, 1H), 7.02-6.90 (m, 4H), 6.42 (d, J = 3 Hz, 1H), 3.88-3.86 (m, 2H), 3.82-3.79 (m, 2H), 2.59 (s, 3H), 2.52 (s, 3H), 2.46 (s, 3H), 2.33 (s, 3H), 2.31 (s, 3H) 1.98 (s, 3H), 1.75-1.56 (m, 21H), 1.11-1.00 (m, 9H), 0.92-0.85 (m, 3H);
¹³C NMR (125 MHz, CDCl₃): d = 249.2, 219.3, 218.7, 138.8, 138.6, 138.4, 138.0, 137.6, 137.5, 136.3, 133.8, 130.4, 130.0, 129.9, 129.1, 128.9, 51.6, 51.2, 35.6, 35.1, 33.1, 33.0, 29.3, 28.9, 27.8, 27.7, 27.6, 27.5, 27.0, 26.5, 26.3, 26.1, 21.2, 21.1, 19.3, 18.7, 18.6, 16.9;
³¹P NMR (202 MHz, CDCl₃): d = 34.2 (s, 1P);
IR (KBr): 2925, 2850, 1813, 1512, 1483, 1430, 1379, 1266, 1169, 1041, 849, 743 cm⁻¹
MS (FD/FI): m/z found for the formula C₄₁H₆₁³⁵ClN₃O₂P¹⁰²Ru: 795.3 (M+).

### Example II:

### Synthesis of a catalyst defined by Formula 1b (according to Scheme I)

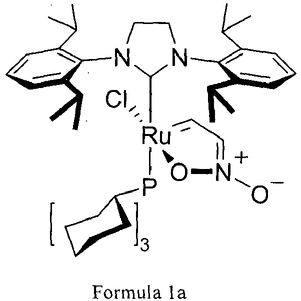

Using a protective argon atmosphere in a Schlenk vessel a solid carbene metal complex defined by Formula **4a,** in which M denotes ruthenium, X¹ and X² denote chlorine, L¹ denotes tricyclohexylphosphine (PCy₃), L² denotes the NHC ligands defined by Formula **2a,** in which R⁶ and R⁹ denote 2,6-di(2-propyl)phenyl, R⁷, R⁸ as well as R¹¹ are hydrogen and R¹² phenyl (149 mg, 0.16 mmol), we added dry deoxygenated dichloromethane (2 ml). Next, we added the compound defined by Formula **3a** (17.4 mg, 0.20 mmol). The resulting solution were mixed at room temperature for about 15 min. From this time, all subsequent operations were performed in the open air, without the need for a protective argon atmosphere. The reaction mixture was concentrated in an evaporator and loaded onto a chromatography column packed with a silica gel. The column was developed with an ethyl acetate-cyclohexane solution (10% v/v), collecting the green fraction. After evaporating off the solvent, we obtained complex **1b** as an olive, microcrystalline solid (93.3 mg, 66% efficiency).
¹H NMR (600 MHz, CDCl₃): d = 13.81 (d, J = 3 Hz, 1H), 7.36-7.10 (m, 6H), 6.29 (d, J = 3 Hz, 1H), 4.20-4.10 (m, 1H), 4.10-4.00 (m, 1H), 4.00-3.80 (m, 3H), 3.75-3.65 (m, 1H), 3.65-3.55 (m, 1H), 2.70-2.64 (m, 1H), 1.70-1.64 (m, 3H), 1.60-1.50 (m, 18H), 1.39-1.35 (m, 3H), 1.26-1.19 (m, 10H), 1.15-1.08 (m, 9H), 1,07-0.92 (m, 14H);
¹³C NMR (150 MHz, CDCl₃): d = 246.4, 222.2, 221.7, 148.64, 148.60, 148.5, 147.4, 137.5, 135.1, 130.0, 129.7, 129.0, 125.2, 124.2, 124.1, 123.9, 77.2, 77.0, 76.8, 54.0, 53.7, 33.2, 33.0, 29.6, 28.7, 28.5, 28.3, 27.9, 27.8, 27.2, 27.2, 26.9, 26.6, 26.3, 26.1, 23.4, 22.8, 22.0;
³¹p NMR (202 MHz, CDCl₃): d = 35.3 (s, 1P);
IR (KBr): 2962, 2927, 2851, 1431, 1414, 1383, 1326, 1269, 1238, 1170, 1047, 803, 758, 734 cm⁻¹;
MS (FD/FI): m/z found for the formula C₄₇H₇₃³⁵ClN₃O₂P¹⁰²Ru: 879.3 (M+).
X-ray structural analysis for compound **1b:**

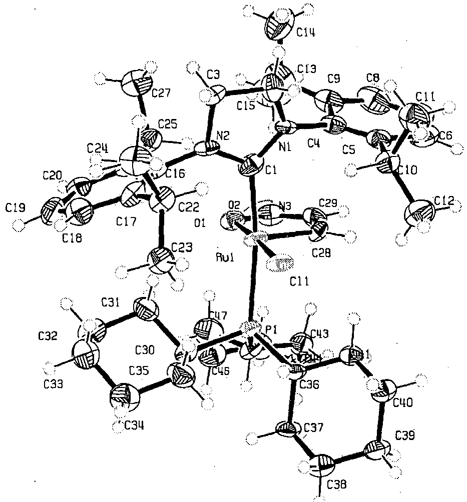

### Example III:

### Synthesis of a catalyst defined by Formula 1c (according to Scheme I)

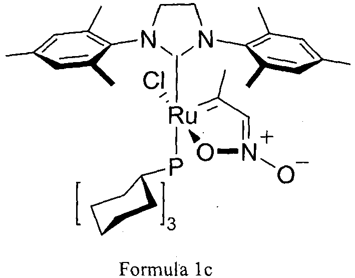

Using a protective argon atmosphere in a Schlenk vessel a solid carbene metal complex defined by Formula **4a,** in which M denotes ruthenium, X¹ and X² denote chlorine, L¹ denotes tricyclohexylphosphine (PCy₃), L² denotes the NHC ligands defined by Formula **2a,** in which R⁶ and R⁹ denote 2,4,6-trimethylphenyl, R⁷, R⁸ as well as R¹¹ are hydrogen and R¹² is phenyl (so-called Grubbs II-generation catalyst, 20.7 mg, 0.024 mmol), we added dry deoxygenated dichloromethane (0.3 ml). Next, we added the compound defined by Formula **3b:**

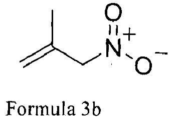

(5 mg, 0.049 mmol). The resulting solution were mixed at room temperature for 20 hours. From this time, all subsequent operations were performed in the open air, without the need for a protective argon atmosphere. The reaction mixture was concentrated in an evaporator and loaded onto a chromatography column packed with a silica gel. The column was developed with an ethyl acetate-cyclohexane solution (10% v/v), collecting the green fraction. After evaporating off the solvent, we obtained complex **1c** as an olive, microcrystalline solid (9.5 mg, 50% efficiency).
¹H NMR (500 MHz, CDCl₃): 7.03 (s, 1H), 6.93 (s, 1H), 6.92 (s, 1H), 6.88 (s, 1H), 6.65 (s, 1H), 4.06-3.97 (m, 1H), 3.88-3.72 (m, 3H), 2.59 (s, 3H), 2.54 (s, 3H), 2.46 (s, 3H), 2.31 (s, 6H), 2.00 (s, 3H), 1.91 (s, 3H), 1.75-1.54 (m, 16H), 1.30-1.00 (m, 15H), 0.92-0.81 (m, 3H);
¹³C NMR (125 MHz, CDCl₃): d = 271.1, 271.0, 217.9, 217.2, 139.0, 138.7, 138.6, 138.3, 138.2, 138.0, 136.6, 133.6, 129.91, 129.85, 129.4, 128.6, 51.9, 51.5, 35.2, 33.6, 33.4, 28.9, 28.8, 27.9, 27.8, 27.6, 27.5, 26.9, 26.5, 21.1, 21.0, 19.2, 18.7, 18.5, 16.6;
³¹P NMR (202 MHz, CDC13): d = 27.0 (s, 1P);
IR (film z CHCl3): 2927, 2851, 1481, 1444, 1268, 1185, 850, 752, 624 cm⁻¹;
MS (FD/FI): m/z found for the formula C₄₂H₆₃³⁵ClN₃O₂P¹⁰²Ru: 809.2 (M+).

### Example IV:

### Synthesis of a catalyst defined by Formula 1d (according to Scheme I)

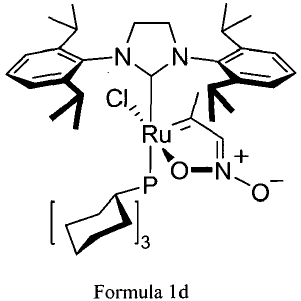

Using a protective argon atmosphere in a Schlenk vessel a solid carbene metal complex defined by Formula **4a,** in which M denotes ruthenium, X¹and X² denote chlorine, L¹ denotes tricyclohexylphosphine (PCy₃), L² denotes the NHC ligands defined by Formula **2a,** in which R⁶ and R⁹ denote 2,6-di(2-propyl)phenyl, R⁷, R⁸ as well as R¹¹ are hydrogen and R¹² phenyl (168 mg, 0.18 mmol), we added dry deoxygenated dichloromethane (2 ml). Next we added the compound defined by Formula **3b** (22.7 mg, 0.23 mmol). The resulting solution were mixed at room temperature for about 15 min. From this time, all subsequent operations were performed in the open air, without the need for a protective argon atmosphere. The reaction mixture was concentrated in an evaporator and loaded onto a chromatography column packed with a silica gel. The column was developed with an ethyl acetate-cyclohexane solution (10% v/v), collecting the green fraction. After evaporating off the solvent, we obtained complex **1d** as an olive, microcrystalline solid (83.1 mg, 52% efficiency).
¹H NMR (600 MHz, CDCl₃): d = 7.40-7.10 (m, 6H), 6.67 (s, 1H), 4.10-4.00 (m, 1H), 3.98-3.87 (m, 2H), 3.68-3.54 (m, 3H), 3.75-3.65 (m, 1H), 3.65-3.55 (m, 1H), 2.41-2.32 (m, 1H), 2.16 (s, 3H), 1.77 (s, 3H), 1.69-1.59 (m), 1.57-1.48 (m), 1.39-1.29 (m), 1.25-1.20 (m), 1.20-1.12 (m), 1.11-1.02 (m), 1.01-0.91 (m).
¹³C NMR (150 MHz, CDCl₃): d = 268.3, 219.5, 219.0, 149.3, 148.8, 148.5, 147.3, 138.1, 130.4, 129.9, 128.9, 124.8, 124.2, 123.2, 77.2, 77.0, 76.8, 55.0, 53.9, 55.4, 35.3, 33.4, 30.9, 29.2, 28.8, 28.6, 28.2, 27.95, 27.88, 27.8, 27.1, 26.95, 26.87, 26.6, 26.4, 26.1, 25.9, 23.9, 23.2, 22.3, 21.7; ³¹P NMR (202 MHz, CDCl₃): d = 26.4 (s, 1P);
IR (film z CHCl3): 2962, 2928, 2851, 1436, 1414, 1268, 1234, 1185, 803, 756, 616 cm⁻¹
MS (FD/FI): m/z found for the formula C₄₉H₇₅³⁵ClN₃O₂P¹⁰²Ru: 893.4 (M⁺).

Examples of uses of compound **1** as catalyst in the metathesis reactions with ring closure, cross-metatheses, "alkene-alkyne" metatheses (ene-yne), as well as the olefin cycloisomerisation reaction.

### Example V:

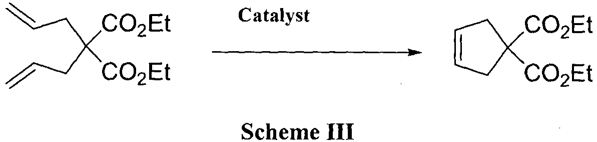

Procedure A: in a Schlenk vessel, we placed a diene solution (48.4 mg, 0.20 mmol) in toluene (2 ml), we added hexachloroethane (1.9 mg, 4%ₘₒₗ), and next, the catalyst **1a** (1.6 mg, 1%ₘₒₗ). The vessel contents were mixed at a temperature of 80°C for 2 h. The raw post-reaction mixture was analysed using gas chromatography. The efficiency of the product metathesis was 100%.

Procedure B: in a Schlenk vessel, we placed a diene solution (48.0 mg, 0.20 mmol) in toluene (2 ml), we added chlorotrimethylsilane (0.9 mg, 4%ₘₒₗ), and next, the catalyst **1a** (1.6 mg, 1%ₘₒₗ). The vessel contents were mixed at a temperature of 80°C for 2 h. The raw post-reaction mixture was analysed using gas chromatography. The efficiency of the product metathesis was 85%.

Procedure C: in a Schlenk vessel, we placed a diene solution (31.2 mg, 0.13 mmol) in carbon tetrachloride (0.6 ml), and next we added catalyst **1b** (5.1 mg, 5%ₘₒₗ). The vessel contents were mixed at a temperature of 60°C for 4 h The raw post-reaction mixture was analysed using gas chromatography. The efficiency of the product metathesis was 98%.

Procedure D: in a Schlenk vessel, we placed a diene solution (30.7 mg, 0.13 mmol) in carbon tetrachloride (0.6 ml), and next we added catalyst **1a** (5.0 mg, 5%ₘₒₗ).The vessel contents were mixed at a temperature of 60°C for 2 h. The raw post-reaction mixture was analysed using gas chromatography. The efficiency of the product metathesis was 100%.

### Example VI:

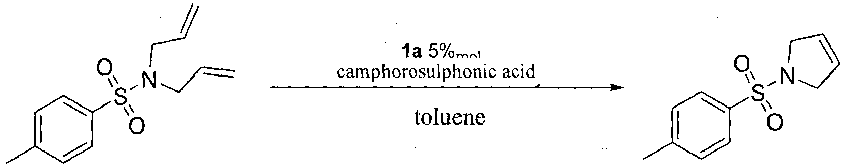

In a Schlenk vessel, we placed a diene solution (74.1 mg, 0.29 mmol) in toluene (1.5 ml), we added camphorosulphonic acid (3.7 mg, 5%ₘₒₗ), and next, the catalyst **1a** (12 mg, 5%ₘₒₗ). The vessel contents were mixed at a temperature of 80°C for 29 h The raw post-reaction mixture was analysed using gas chromatography. The conversion was 99%.

### Example VII:

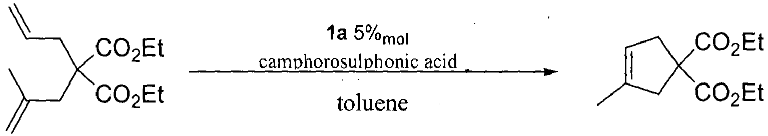

In a Schlenk vessel, we placed a diene solution (77.9 mg, 0.31 mmol) in toluene (1.5 ml), we added camphorosulphonic acid (5.1 mg, 7%ₘₒₗ), and next, the catalyst **1a** (11.9 mg, 5%ₘₒₗ). The vessel contents were mixed at a temperature of 80°C for 14 h The raw post-reaction mixture was analysed using gas chromatography. The conversion was 100%.

### Example VIII:

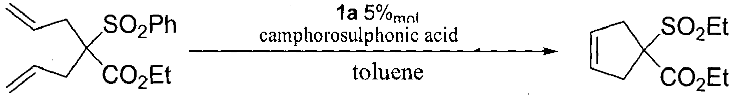

In a Schlenk vessel, we placed a diene solution (92.7 mg, 0.31 mmol) in toluene (1.5 ml), we added camphorosulphonic acid (4.4 mg, 6%ₘₒₗ), and next, the catalyst **1a** (12.0 mg, 5%ₘₒₗ). The vessel contents were mixed at a temperature of 80°C for 2 h. The raw post-reaction mixture was analysed using gas chromatography. The conversion was 100%.

### Example IX:

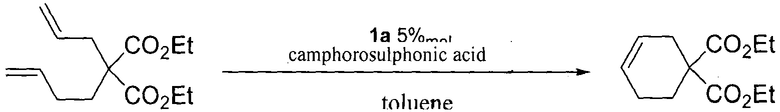

In a Schlenk vessel, we placed a diene solution (76.0 mg, 0.31 mmol) in toluene (1.5 ml), we added camphorosulphonic acid (4.6 mg, 7%ₘₒₗ), and next, the catalyst la (11.9 mg, 5%ₘₒₗ). The vessel contents were mixed at a temperature of 80°C for 3 h The raw post-reaction mixture was analysed using gas chromatography. The conversion was 100%.

### Example X:

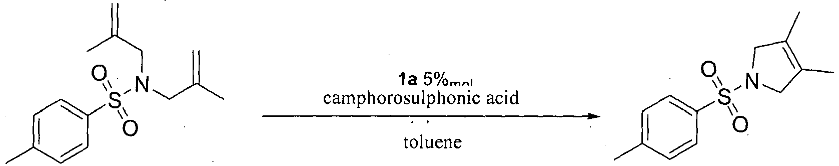

In a Schlenk vessel, we placed a diene solution (83.4 mg, 0.30 mmol) in toluene (1.5 ml), we added camphorosulphonic acid (3.8 mg, 5%ₘₒₗ), and next, the catalyst **la** (11.9 mg, 5%ₘₒₗ). The vessel contents were mixed at a temperature of 80°C for 53 h The raw post-reaction mixture was analysed using gas chromatography. The conversion was 84%.

### Example XI:

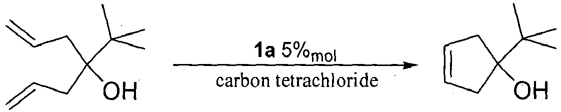

In a Schlenk vessel, we placed a diene solution (50.3 mg, 0.30 mmol) in carbon tetrachloride (1.5 ml), and next we added catalyst **la** (12.2 mg, 5%ₘₒₗ)_{.} The vessel contents were mixed at a temperature of 65°C for 3 h The raw post-reaction mixture was analysed using gas chromatography. The conversion was 100%.

### Example XII:

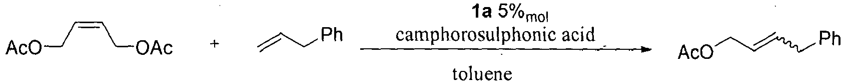

In a Schlenk vessel, we placed roztwór diacetoxybutenu (110.0 mg, 0.64 mmol) and allylbenzene (35.8 mg, 0.30 mmol) in toluene (1.5 ml), we added camphorosulphonic acid (4.7 mg, 7%ₘₒₗ), and next, the catalyst la (12.1 mg, 5%ₘₒₗ). The vessel contents were mixed at a temperature of 80°C for 29 h The raw post-reaction mixture was analysed using gas chromatography. The efficiency of the product metathesis was 41%.

### Example XIII:

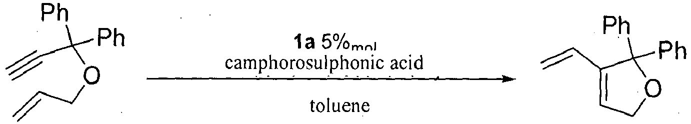

In a Schlenk vessel, we placed an enyne solution (76.1 mg, 0.31 mmol) in toluene (1.5 ml), we added camphorosulphonic acid (4.5 mg, 6%ₘₒₗ), and next, the catalyst **la** (12.4 mg, 5%ₘₒₗ). The vessel contents were mixed at a temperature of 80°C for 24 h The raw post-reaction mixture was analysed using gas chromatography. The conversion was 100%.

### Example XIV:

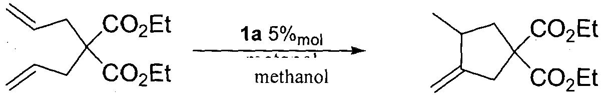

In a Schlenk vessel, we placed a diene solution (73.5 mg, 0.31 mmol) in methanol (1.5 ml), and next we added catalyst **la** (11.7 mg, 5%ₘₒₗ). The vessel contents were mixed at a temperature of 65°C for 42 h The raw post-reaction mixture was analysed using gas chromatography. The efficiency of the product cycloisomerisation was 82%.

### Example XV:

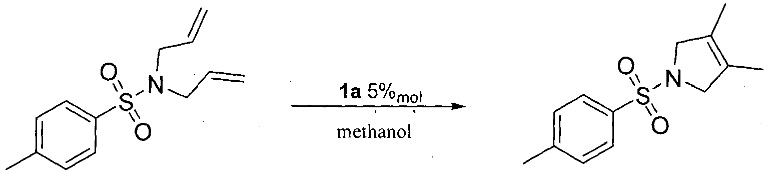

In a Schlenk vessel, we placed a diene solution (77.4 mg, 0.31 mmol) in methanol (1.5 ml), and next we added catalyst **1a** (11.9 mg, 5%ₘₒₗ). The vessel contents were mixed at a temperature of 65°C for 50 h The raw post-reaction mixture was analysed using gas chromatography. The efficiency of the product cycloisomerisation was 88%.

### Example XVI:

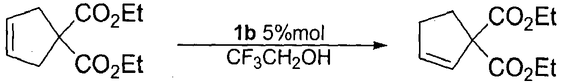

In a Schlenk vessel, we placed an olefin solution (54.1 mg, 0.25 mmol) in trifluoroethanol (2 ml), and next we added catalyst **1a** (10.8 mg, 5%ₘₒₗ). The vessel contents were mixed at a temperature of 65 °C for 71 h The raw post-reaction mixture was analysed using gas chromatography. The efficiency of the product isomerisation was 77%.

### Example XVII:

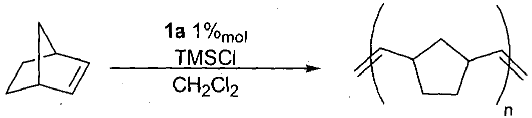

In a Schlenk vessel, we placed a norbornene solution (187 mg, 1.4 mmol) in dichloromethane (5 ml) and were mixed at a temperature of 40 °C. Next, we added chlorotrimethylsilane (6.1 mg, 4%ₘₒₗ) and catalyst **1a** (11.1 mg, 1%ₘₒₗ). The vessel contents were mixed at the same temperature for 10 min, whereafter this was poured into another vessel containing 15 ml of methanol and a white solid was precipitated which was filtered out and dried under reduced pressure over a vacuum pump. We obtained a product (119 mg, 90% efficiency) in the form of a white solid.

### Example XVIII:

Production of polidicyclopentadiene: a flask was loaded with dicyclopentadiene (132 mg, 1.0 mmol) in toluene (5 mL) and mixed at room temperature. Next, we added a chlorotrimethylsilane solution (1.1 mg, 1%ₘₒₗ) and catalyst **1a** (0.2 mg, 0.025%ₘₒₗ in toluene and the flask contents were mixed at the same temperature for 10 min. Next, we supplemented the flask with toluene and brought it to boiling temp. in order to wash off the unreacted dicyclopentadiene. The insoluble polymer was washed with toluene and dried under reduced pressure at a temperature of 100 °C for 12 h The conversion of dicyclopentadiene was 99%.

### Example XIX:

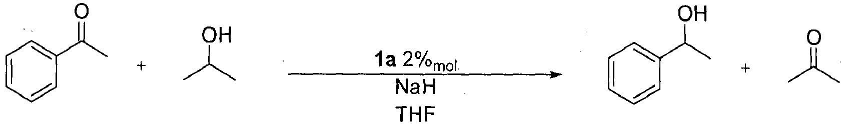

In a Schlenk vessel, we placed a solution of catalyst **1a** (15.7 mg, 2%ₘₒₗ) in tetrahydrofuran (2.5 ml) and we added sodium hydride (2.8 mg, 7%ₘₒₗ). To this mixture we then added acetophenone (120.3 mg, 1.0 mmol) and isopropyl alcohol (2.5 ml). The vessel contents were mixed at a temperature of 70 °C for 5 h. The raw mixture was purified using column chromatography on a silica gel (elution with cyclohexane: ethyl acetate 20:1). We obtained 95 mg of a liquid product (efficiency 78%).

## Claims

1. A metal complex defined by Formula **1**: 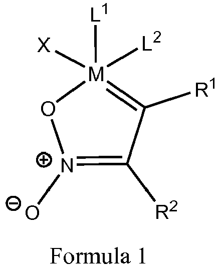 in which:
M denotes ruthenium;
X denotes a chlorine atom;
R¹ denotes a hydrogen atom, methyl group;
R² denotes a hydrogen atom;
neutral ligand L¹ denotes -P(R⁵)₃ in which substituent R⁵ denotes, independently of one another, C₁-C₁₂ alkyl, C₃-C₁₂ cycloalkyl, C₅-C₂₀ aryl, 5-12 membered heteroaryl; and
neutral ligand L² denotes ligands defined by Formula **2a** or **2b**: 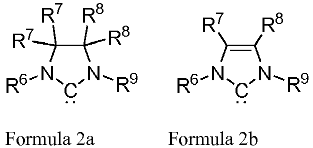 in which substituents R⁶, R⁷, R⁸ and R⁹ denotes, independently of one another, a hydrogen atom, C₁-C₁₂ alkyl, C₃-C₁₂ cycloalkyl, C₂-C₁₂ alkenyl or C₅-C₂₀ aryl which may be substituted with at least one C₁-C₁₂ alkyl, C₁-C₁₂ perfluoroalkyl, C₁-C₁₂ alkoxyl or halogen atom, and groups _{R}⁶, _{R}⁷, R⁸, R⁹ and R¹⁰ may possibly be interconnected.

2. A method of producing a the ruthenium complex defined in Claim 1, **characterised in that** the compound defined by Formula **3** 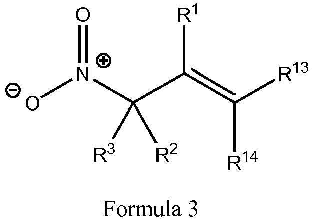 in which R¹, R² denote, independently of one another, a hydrogen atom, a fluoride atom, C₁-C₂₅ alkyl, C₁-C₂₅ perfluoroalkyl, C₂-C₂₅ alkene, C₃-C₇ cycloalkyl, C₂-C₂₅ alkenyl, C₃-C₂₅ cycloalkenyl, C₂-C₂₅ alkynyl, C₃-C₂₅ cycloalkynyl, C₁-C₂₅ alkoxyl, C₅-C₂₄ aryl, heteroaryl C₅-C₂₀, or a 3-12 membered heterocycle wherein the alkyl groups may be joined together in a ring, preferentially a hydrogen, a nitro group (-NO₂), a cyanide group (-CN), carboxyl (-COOH), ester (-COOR'), amido (-CONR'₂), sulphonyl (-SO₂R^{'}), formyl (-CHO), sulphonoamido (-SO₂NR'₂), ketone (-COR'), in which R has the following meaning: C₁-C₅ alkyl, C₁-C₅ perfluoroalkyl, C₅-C₂₄ aryl, whereas R³, R¹³ ,R¹⁴ denote, independently of one another, a hydrogen atom, a fluoride atom, a C₁-C₂₅ alkyl, C₁-C₂₅ perfluoroalkyl, C₂-C₂₅ alkene, C₃-C₇ cycloalkyl, C₂-C₂₅ alkenyl, C₃-C₂₅ cycloalkenyl, C₂-C₂₅ alkynyl, C₃-C₂₅ cycloalkynyl, C₁-C₂₅ alkoxyl, C₅-C₂₄ aryl, heteroaryl C₅-C₂₀, or a 3-12 membered heterocycle wherein the alkyl groups may be joined together in a ring, preferentially a hydrogen, a nitro group (-NO₂), a cyanide group (-CN), a carboxyl (-COOH), ester (-COOR'), amido (-CONR'₂), sulphonyl (-SO₂R^{'}), formyl (-CHO), sulphonoamido (-SO₂NR'₂), or ketone (-COR') group, in which R has the following meaning: C₁-C₅ alkyl, C₁-C₅ perfluoroalkyl or C₅-C₂₄ aryl;
is reacted with carbene complexes of ruthenium defined by Formula **4a, 4b, 4c or 4d**: 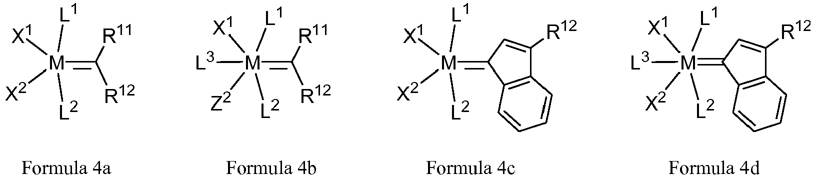 in which
M denotes ruthenium or osmium;
L¹ L² and L³, independently of one another, denote neutral ligands;
X¹ and X², independently of one another, denote an anionic ligand;
R¹¹ has the same meaning as R¹ of Formula **1**;
R¹² denotes a hydrogen atom, C₅-C₂₀ aryl, C₅-C₂₀ heteroaryl, vinyl or allenyl.

3. The method according to Claim 2, **characterised in that** the reaction is carried out over a period from 1 min. to 250 h, at a temperature of from 0 to 150 °C.

4. The method according to Claim 2 or 3, **characterised in that** the reaction is carried out in a protic or aprotic solvent, a chlorinated solvent or in an aromatic hydrocarbon solvent, or in mixtures thereof.

5. The method according to any of Claim from 2 to 4, **characterised in that** the reaction is carried out in a solvent selected from among methylene chloride and/or toluene.

6. A use the ruthenium complex defined by Formula **1** defined in Claim 1, as a (pre)catalyst in metathesis processes, isomerisation and cycloisomerisation of olefins as well as in of the hydrogen transfer reaction.

7. The use according to Claim 6, **characterised in that** complexes of ruthenium are used as (pre)catalysts in ring closing metathesis reactions, homometathesis, cross-metathesis, "alkene-alkyne" metathesis (ene-yne) or in ROMP polymerisation reactions.

8. The use according to Claim 7, **characterised in that** complexes of ruthenium are used as (pre)catalysts in a metathetic polymerisation with dicyclopentadiene ring opening.

9. The use according to Claim 6 or 7, **characterised in that** the reaction is carried out in the presence of an acid or halide derivatives of alkanes and silanes or *N*-haloimides and amides.

## Patentansprüche

1. Metallkomplex, definiert durch Formel **1**: 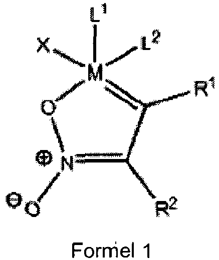 worin:
M Ruthenium bezeichnet;
X ein Chloratom bezeichnet;
R¹ ein Wasserstoffatom, eine Methylgruppe bezeichnet;
R² ein Wasserstoffatom bezeichnet;
Neutraler Ligand L¹ -P(R⁵)₃ bezeichnet, worin der Substituent R⁵ unabhängig voneinander C₁-C₁₂ Alkyl, C₃-C₁₂ Cycloalkyl, C₅-C₂₀ Aryl, ein 5-12 Mitglieder zählendes Heteroaryl bezeichnet; und Neutraler Ligand L² durch die Formel **2a** oder **2b** definierte Liganden bezeichnet: 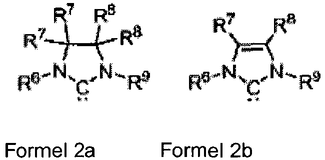 worin die Substituenten R⁶, R⁷, R⁸ und R⁹ unabhängig voneinander ein Wasserstoffatom, C₁-C₁₂ Alkyl, C₃-C₁₂ Cycloalkyl, C₂-C₁₂ Alkenyl oder C₅-C₂₀ Aryl bezeichnen, die mit mindestens einem C₁-C₁₂ Alkyl, C₁-C₁₂ Perfluoralkyl, C₁-C₁₂ Alkoxyl oder Halogenatom substituiert sein können, und wobei die Gruppen R⁶, _{R}⁷, R⁸, R⁹ und R¹⁰ miteinander verbunden sein können.

2. Verfahren zur Herstellung des Rutheniumkomplexes nach Anspruch 1, **dadurch gekennzeichnet, dass** die durch Formel **3** definierte Verbindung 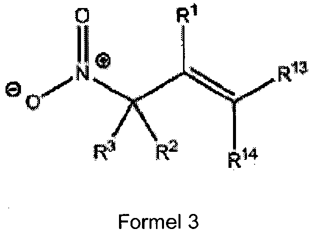 in der R¹, R² unabhängig voneinander ein Wasserstoffatom, ein Fluoridatom, C₁-C₂₅ Alkyl, C₁-C₂₅ Perfluoralkyl, C₂-C₂₅ Alken, C₃-C₇ Cycloalkyl, C₂-C₂₅ Alkenyl, C₃-C₂₅ Cycloalkenyl, C₂-C₂₅ Alkinyl, C₃-C₂₅ Cycloalkinyl, C₁-C₂₅ Alkoxyl, C₅-C₂₄ Aryl, Heteroaryl C₅-C₂₀, oder einen 3-12 Mitglieder zählenden Heterocyclus bezeichnen, worin die Akylgruppen in einem Ring zusammengefügt sein können, bevorzugt ein Wasserstoff, eine Nitrogruppe (-NO₂), eine Cyanidgruppe (-CN), Carboxyl (-COOH), Ester (-COOR'), Amido (-CONR'₂), Sulphonyl (-SO₂R') Formyl (-CHO), Sulphonamido (-SO₂NR'₂), Keton (-COR'), worin R' die folgende Bedeutung hat: C₁-C₅ Alkyl, C₁-C₅ Perfluoralkyl, C₅-C₂₄ Aryl, wohingegen R³, R¹³, R¹⁴ unabhängig voneinander ein Wasserstoffatom, ein Fluoridatom, ein C₁-C₂₅ Alkyl, C₁-C₂₅ Perfluoralkyl, C₂-C₂₅ Alken, C₃-C₇ Cycloalkyl, C₂-C₂₅ Alkenyl, C₃-C₂₅ Cycloalkenyl, C₂-C₂₅ Alkinyl, C₃-C₂₅ Cycloalkinyl, C₁-C₂₅ Alkoxyl, C₅-C₂₄ Aryl, Heteroaryl C₅-C₂₀ oder einen 3-12 Mitglieder zählenden Heterocyclus bezeichnen, worin die Akylgruppen in einem Ring zusammengefügt sein können, bevorzugt ein Wasserstoff, eine Nitrogruppe (-NO₂) eine Cyanidgruppe (-CN), Carboxyl- (-COOH), Ester- (-COOR'), Amido- (-CONR'₂), Sulphonyl- (-SO₂R'), Formyl- (-CHO), Sulphonamido- (-SO₂NR'₂) oder Keton- (-COR') gruppe, worin R' die folgende Bedeutung hat: C₁-C₅ Alkyl, C₁-C₅ Perfluoralkyl oder C₅-C₂₄ Aryl;
reagiert wird mit Carbenkomplexen von Ruthenium, wie definiert durch Formel **4a, 4b, 4c oder 4d**: 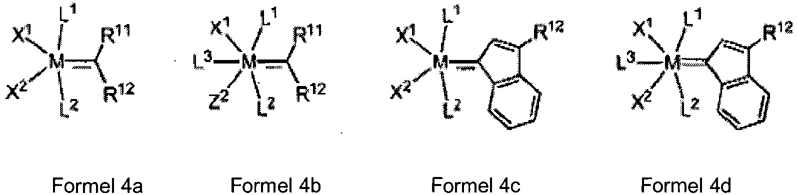 worin
M Ruthenium oder Osmium bezeichnet;
L¹, L² und L³ unabhängig voneinander neutrale Liganden bezeichnen;
X¹ und X² unabhängig voneinander einen anionischen Liganden bezeichnen;
R¹¹ dieselbe Bedeutung hat wie R¹ in Formel **1**;
R¹² ein Wasserstoffatom, C₅-C₂₀ Aryl, C₅-C₂₀ Heteroaryl, Vinyl oder Allenyl bezeichnet.

3. Verfahren nach Anspruch 2, **dadurch gekennzeichnet, dass** die Reaktion über eine Zeitdauer von 1 min. bis 250 h bei einer Temperatur von 0 bis 150°C durchgeführt wird.

4. Verfahren nach Anspruch 2 oder 3, **dadurch gekennzeichnet, dass** die Reaktion in einem protischen oder aprotischen Lösungsmittel, einem chlorinierten Lösungsmittel oder in einem aromatischen Kohlenwasserstoff-Lösungsmittel oder in Gemischen davon durchgeführt wird.

5. Verfahren nach einem der Ansprüche von 2 bis 4, **dadurch gekennzeichnet, dass** die Reaktion in einem Lösungsmittel ausgewählt aus Methylenchlorid und/oder Toluol durchgeführt wird.

6. Verwendung des Rutheniumkomplexes wie definiert durch Formel 1 in Anspruch 1 als ein (Vor)Katalysator in Metatheseprozessen, der Isomerisierung und Cycloisomerisierung von Olefinen, sowie in Wasserstoff-Transferreaktionen.

7. Verwendung nach Anspruch 6, **dadurch gekennzeichnet, dass** die Rutheniumkomplexe als (Vor)Katalysatoren in Ringschluss-Metathesereaktionen, Homometathese, Kreuz-Metathesw, "Alken-Alkin" Metathese (en-in), oder in ROMP Polymerisationsreaktionen verwendet werden.

8. Verwendung nach Anspruch 7, **dadurch gekennzeichnet, dass** die Rutheniumkomplexe als (Vor)Katalysatoren in einer Metathese-Polymerisation mit einer Dicyclopentadien-Ringöffnung verwendet werden.

9. Verwendung nach Anspruch 6 oder 7, **dadurch gekennzeichnet, dass** die Reaktion in Anwesenheit einer Säure oder Halidderivaten von Alkanen und Silanen oder *N*-Haloimiden und Amiden durchgeführt wird.

## Revendications

1. Complexe métallique défini par la formule 1 : 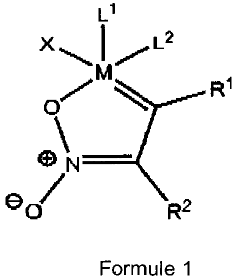 dans laquelle :
M indique le ruthénium ;
X indique un atome de chlore ;
R¹ indique un atome d'hydrogène, un groupe méthyle ;
R² indique un atome d'hydrogène ;
le ligand neutre L¹ indique -P(R⁵)₃ dans lequel chaque substituant R⁵ indique, indépendamment les uns des autres, un alkyle en C₁-C₁₂, un cycloalkyle en C₃-C₁₂, un aryle en C₅-C₂₀, un hétéroaryle à 5-12 chaînons ; et
le ligand neutre L² indique des ligands définis par la formule **2a** ou **2b** : 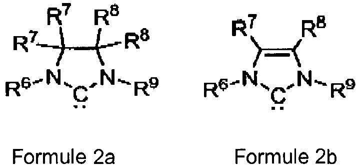 dans lesquelles les substituants R⁶, R⁷, R⁸ et R⁹ indiquent, indépendamment les uns des autres, un atome d'hydrogène, un alkyle en C₁-C₁₂, un cycloalkyle en C₃-C₁₂, un alcényle en C₂-C₁₂ ou un aryle en C₅-C₂₀ qui peut être substitué par au moins un alkyle en C₁-C₁₂, un perfluoroalkyle en C₁-C₁₂, un alcoxyle en C₁-C₁₂ ou un atome d'halogène, et les groupes R⁶, R⁷, R⁸, R⁹ et R¹⁰ peuvent éventuellement être interconnectés.

2. Procédé de production du complexe de ruthénium défini dans la revendication 1, **caractérisé en ce que** le composé défini par la formule 3 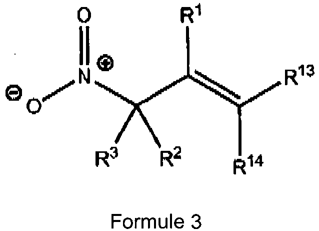 dans laquelle R¹, R² indiquent, indépendamment les uns des autres, un atome d'hydrogène, un atome de fluorure, un alkyle en C₁-C₂₅, un perfluoroalkyle en C₁-C₂₅, un acène en C₂-C₂₅, un cycloalkyle en C₃-C₇, un alcényle en C₂-C₂₅, un cycloalcényle en C₃-C₂₅, un alcynyle en C₂-C₂₅, un cycloalcynyle en C₃-C₂₅, un alcoxyle en C₁-C₂₅, un aryle en C₅-C₂₄, un hétéroaryle en C₅-C₂₀ ou un hétérocycle à 3-12 chaînons dans lequel les groupes alkyle peuvent être assemblés ensemble dans un cycle, de préférence un hydrogène, un groupe nitro (-NO₂), un groupe cyanure (-CN), carboxyle (-COOH), ester (-COOR'), amido (CONR'₂), sulfonyle (-SO₂R'), formyle (-CHO), sulfonamido (-SO₂NR'₂), cétone (-COR'), dans lesquels R' a la signification suivante: alkyle en C₁-C₅, perfluoroalkyle en C₁-C₅, aryle en C₅-C₂₄, tandis que R³, R¹³, R¹⁴ indiquent, indépendamment les uns des autres, un atome d'hydrogène, un atome de fluorure, un alkyle en C₁-C₂₅, un perfluoroalkyle en C₁-C₂₅, un alcène en C₂-C₂₅, un cycloalkyle en C₃-C₇, un alcényle en C₂-C₂₅, un cycloalcényle en C₃-C₂₅, un alcynyle en C₂-C₂₅, un cycloalcynyle en C₃-C₂₅, un alcoxyle en C₁-C₂₅, un aryle en C₅-C₂₄, un hétéroaryle en C₅-C₂₀, ou un hétérocycle à 3-12 chaînons dans lesquels les groupes alkyle peuvent être assemblés ensemble dans un cycle, de préférence un hydrogène, un groupe nitro (-NO₂), un groupe cyanure (-CN), carboxyle (-COOH), ester (-COOR'), amido (CONR'₂), sulfonyle (-SO₂R'), formyle (-CHO), sulfonamido (-SO₂NR'₂ ou cétone (-COR'), dans lesquels R' a la signification suivante : alkyle en C₁-C₅, perfluoroalkyle en C₁-C₅ ou aryle en C₅-C₂₄ ;
est mis à réagir avec des complexes carbène de ruthénium définis par les formules **4a, 4b, 4c ou 4d** : 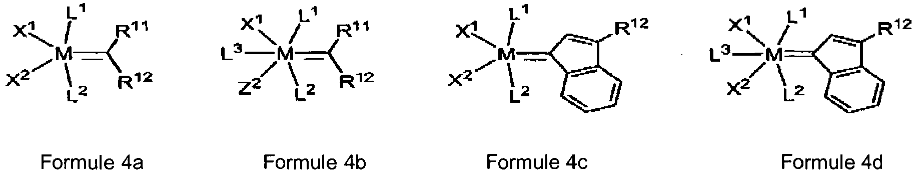 dans lesquelles
M indique le ruthénium ou l'osmium ;
L¹, L² et L³, indépendamment les uns des autres, indiquent des ligands neutres ;
X¹ et X², indépendamment l'un de l'autre, indiquent un ligand anionique ;
R¹¹ a la même signification que R¹ de la formule **1** ;
R¹² indique un atome d'hydrogène, un aryle en C₅-C₂₀, un hétéroaryle en C₅-C₂₀, un vinyle ou un allényle.

3. Procédé selon la revendication 2, **caractérisé en ce que** la réaction est réalisée sur une période de 1 min à 250 h, à une température de 0 à 150°C.

4. Procédé selon la revendication 2 ou 3, **caractérisé en ce que** la réaction est réalisée dans un solvant protique ou aprotique, un solvant chloré ou dans un solvant hydrocarboné aromatique, ou dans des mélanges de ceux-ci.

5. Procédé selon l'une quelconque des revendications 2 à 4, **caractérisé en ce que** la réaction est réalisée dans un solvant sélectionné parmi le chlorure de méthylène et/ou le toluène.

6. Utilisation du complexe de ruthénium défini par la formule **1** définie dans la revendication 1 en tant que (pré)catalyseur dans des processus de métathèse, l'isomérisation et la cycloisomérisation d'oléfines ainsi que dans la réaction de transfert d'hydrogène.

7. Utilisation selon la revendication 6, **caractérisée en ce que** des complexes de ruthénium sont utilisés en tant que (pré)catalyseurs dans des réactions de métathèse de fermeture de cycle, d'homométathèse, de métathèse croisée, de métathèse « d'alcène-alcyne » (ène-yne) ou dans des réactions de polymérisation ROMP.

8. Utilisation selon la revendication 7, **caractérisée en ce que** des complexes de ruthénium sont utilisés en tant que (pré)catalyseurs dans une polymérisation par métathèse avec une ouverture de cycle dicyclopentadiène.

9. Utilisation selon la revendication 6 ou 7, **caractérisée en ce que** la réaction est réalisée en présence d'un acide ou de dérivés halogénés d'alcanes et de silanes ou de *N*-halogénoimides et d'amides.
